(19) Europäisches Patentamt
European Patent Office
Office européen des brevets

(11) EP 1 007 702 B1

(12) # EUROPÄISCHE PATENTSCHRIFT

(45) Veröffentlichungstag und Bekanntmachung des Hinweises auf die Patenterteilung:
**17.08.2005 Patentblatt 2005/33**

(21) Anmeldenummer: **98949962.9**

(22) Anmeldetag: **20.08.1998**

(51) Int Cl.[7]: **C12N 15/81**, C12N 15/54, C12N 9/10, C12P 19/02, C12P 19/12, C12P 19/28

(86) Internationale Anmeldenummer:
**PCT/EP1998/005309**

(87) Internationale Veröffentlichungsnummer:
**WO 1999/010511 (04.03.1999 Gazette 1999/09)**

(54) **VERFAHREN ZUR ERHÖHUNG DER GENEXPRESSION VON SACCHAROSE SYNTHASE**
METHOD FOR INCREASING THE GENE EXPRESSION OF SACCHAROSE SYNTHASE
PROCEDE D'AUGMENTATION DE L'EXPRESSION DU GENE DE LA SACCHAROSE SYNTHASE

(84) Benannte Vertragsstaaten:
**BE CH DE DK FR GB IT LI NL SE**

(30) Priorität: **21.08.1997 DE 19736343**

(43) Veröffentlichungstag der Anmeldung:
**14.06.2000 Patentblatt 2000/24**

(73) Patentinhaber: **Forschungszentrum Jülich GmbH 52425 Jülich (DE)**

(72) Erfinder:
- **FROMMER, Wolf-Bernd D-72072 Tübingen (DE)**
- **SCHRADER, Henning D-52382 Niederzier (DE)**
- **ELLING, Lothar D-52066 Aachen (DE)**

(56) Entgegenhaltungen:
**EP-A- 0 284 044**
**EP-A- 0 717 107**
**WO-A-94/00574**
**WO-A-94/01540**

- **MELLOR J. ET AL.: "Factors affecting heterologous gene expression in saccharomyces cerevisiae" GENE, Bd. 33, 1985, Seiten 215-226, XP002086158**
- **JAUNIAUX J.-C. ET AL.: "Nitrogen catabolite regulation of proline permease in Saccharomyces cerevisiae. Cloning of the PUT4 gene and study of PUT4 RNA levels in wild-type and mutant strains" EUROPEAN JOURNAL OF BIOCHEMISTRY, Bd. 164, Nr. 3, 1. Mai 1987, Seiten 601-606, XP002086140 in der Anmeldung erwähnt**
- **GIETZ R.D.; SUGINO A.: 'New yeast-Escherichia coli shuttle vectors constructed with in vitro mutagenized yeast genes lacking six-base pair restriction sites' GENE Bd. 74, 1988, Seiten 527 - 534**
- **RENTSCH D. ET AL: 'NTR1 encodes a high affinity oligopeptide transporter in Arabidopsis' FEBS LETTERS Bd. 370, 1995, Seiten 264 - 268**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 007 702 B1

Printed by Jouve, 75001 PARIS (FR)

## Beschreibung

Die Erfindung betrifft ein Verfahren zur Erhöhung der Genexpression von Saccharose Synthase gemäß den Ansprüchen 1 bis 8, ein Saccharose Synthasegen gemäß Anspruch 9, Genstrukturen nach Anspruch 10, Vektoren nach Anspruch 11 sowie transformierte Zellen nach Anspruch 12 bis 15.

Die Saccharose Synthase ist ein auf Pflanzen beschränktes Enzym. Von vielen höheren Pflanzen sind inzwischen je 2 unterschiedliche Gene pro Art bekannt; beim Reis kommen sogar 3 unterschiedliche Gene vor. In ihren biochemischen Eigenschaften nur wenig unterschiedlich, dienen sie mit unterschiedlichen Promotoren ausgestattet vor allem einer exakten Steuerung der Enzymexpression in unterschiedlichen Entwicklungsstadien und Organen der Pflanze. So kommt im Reis 8 Tage nach der Keimung die Saccharose Synthase 1 besonders in Wurzel und Stengel vor, während die Saccharose Synthase 2 in der gesamten Pflanze verteilt vorkommt. Saccharose Synthase 3 ist besonders in sich füllenden Reiskörnern aktiv, also in einer ganz anderen Lebensphase der Pflanze.

In der Pflanze dient das Enzym der Spaltung von Saccharose gemäß folgender Gleichung, wobei *in vivo* ausschließlich die Spaltreaktion Bedeutung hat:

$$\text{Saccharose} + \text{UDP} \underset{\text{Synthesereaktion}}{\overset{\text{Spaltreaktion}}{\rightleftharpoons}} \text{UDP-Glucose} + \text{Fructose}$$

Die Saccharose als Transportform der Kohlenhydrate in Pflanzen wird so in ihren Zielzellen, wie Zellen der Speicherorgane, Samen, sich entwickelnde Pflanzenorgane, in die direkt weiter nutzbare UDP-Glucose und Fructose gespalten. Während die Fructose für weitere Nutzungen zunächst umgelagert werden muß, steht die UDP-Glucose direkt für die Synthese von Stärke und Cellulose zur Verfügung.

Von wirtschaftlichem und wissenschaftlichem Interesse sind sowohl Spalt- wie Syntheserichtung des Enzyms. In Syntheserichtung wird eine Vielzahl von räumlich ähnlich aufgebauten Zuckern und Nichtzuckern akzeptiert. In Spaltrichtung werden Derivate der Saccharose akzeptiert, außerdem bevorzugt die Nucleosiddiphosphate (NDPs) UDP, ADP und TDP.

Somit wird eine große Zahl von saccharoseanalogen Disacchariden zugänglich. Diese sind interessant zur Erforschung von Struktur und Funktion von Glykokonjugaten, wie Glykolipiden und Glykoproteinen. Da Glykokonjugate auch an der Kommunikation der Zellen untereinander beteiligt sind, haben viele dieser Strukturen auch eine Bedeutung in der medizinischen Forschung.

Gewinnbar ist das Enzym aus unterschiedlichen pflanzlichen Quellen. Für die Aufarbeitung beispielsweise aus Reissamen müssen die Samen zunächst gequollen werden; anschließend werden sie aufgeschlossen, die festen Bestandteile weitgehend abfiltriert und das Filtrat an einer Ionenaustauschersäule vorgereinigt. Das nach dem Säulenschritt große Volumen wird eingeengt und einer Gelfiltration unterworfen. Deren aktive Fraktionen sind für Synthesen verwendbar (vgl. dazu auch DE 4 221 595).

Die beschriebene Gewinnung der Saccharose Synthase aus Reissamen ist insofern problematisch, als Reissamen nur eine geringe Aktivität von 0,56 Units pro Gramm Trockengewicht besitzen. Bei der Aufreinigung störend sind außerdem die reichlich vorhandenen Kohlenhydrate in Form von Stärke und Cellulose. Weiterhin konnte das störende Enzym Invertase, das Saccharose spaltet, nicht vollständig abgetrennt werden. Eine Reduktion des außerdem störenden Enzyms Phosphatase, das NDPs zersetzt, und der Nucleotidzucker spaltenden Enzyme, die NDP-Zukker zersetzen, wäre sinnvoll. Darüber hinaus ist von Nachteil, daß die Saccharose Synthase im Reis offenbar nicht als reines Enzym, sondern in wechselnden Verhältnissen von Isoenzymen vorliegt, die offensichtlich unterschiedliche Syntheseeigenschaften besitzen. Einige Synthesen von Disacchariden lassen sich deshalb mit unterschiedlichen Enzymchargen nur schwer reproduzieren. Da die Isoenzyme unterschiedlichen Reaktionskinetiken folgen, wurden Messungen der Kinetiken erschwert. Weiterhin lassen sich die Isoenzyme nicht voneinander trennen, da sie einander sehr ähnlich sind. Schließlich ist die Aufreinigung auch sehr langwierig, da sich insbesondere auf die Gelfiltrationssäule nur kleine Fraktionen laden lassen. Eine Verkürzung der Reinigung wäre daher wünschenswert.

Zur Vermeidung der oben angeführten Nachteile ist die Schaffung eines rekombinanten mikrobiellen Systems die Methode der Wahl. Um damit auch größere Mengen des Enzyms kostengünstig und umweltfreundlich produzieren zu können, sollten teure Zusatzstoffe oder Gifte im Anzuchtmedium vermieden werden. Ferner sollte nur ein einziges Saccharose Synthasegen zur Expression gelangen. Im Sinne einer beschleunigten Aufreinigung sollten Expressionen und / oder Aktivitäten von störenden Enzymen möglichst gering sein.

Es wurde daher bereits der Versuch unternommen, ein Saccharose Synthasegen aus Solanum tuberosum (vgl. Salanoubat, M. und Belliard, G.: Molecular cloning and sequencing of sucrose synthase cDNA from potato (Solanum tuberosum L.): preliminory characterization of sucrose synthase mRNA distribution, in Gene 60, 47 - 56, 1987) in dem Saccharomyces cerevisiae - Stamm YSH zu exprimieren (vgl. Riesmeier, J.W. et. al.: Isolation and characterization of a sucrose carrier cDNA from spinach by functional expression in yeast. EMBO J. 11 (13), 4705-4713, (1992). Dafür wurde das Gen unter der Kontrolle eines ADH-Promotors in das Plasmid 128A2 kloniert (vgl. Figur 1) und anschließend in den genannten Hefestamm transformiert. Der ADH-Promotor regelt normalerweise die Ablesehäufigkeit des Hefeenzyms Alkoholdehydrogenase, das konstitutiv exprimiert wird und daher stets in ungefähr gleichen Mengen im Organismus vorhanden ist. Somit sollte also auch die Saccharose Synthase unter Kontrolle dieses Promotors ähnliche Expressionseigenschaften besitzen.

Die erzielte Expression verlieh dem Hefestamm zwar die Fähigkeit, Saccharose zu spalten. Jedoch war die spezifische Aktivität des Enzyms relativ gering. Je nach Bestimmungsmethode wurden 5 bis 25 mU Saccharose Synthase / mg Protein bestimmt.

Die Veröffentlichung " New yeast- Escherichia coli shuttle vectors constructed with in vito mutagenized yeast genes lacking six-base pair restriction sites" (Gene, 74, (1988) 527 - 534); R. Daniel Gietz, Akio Sugino) offenbart die Produktion von Allelen von LEU2, URA3 und TRP1 Genen von Saccharomyces cerevisiae durch in vito Mutagenese. Die Allele werden verwendet um Hefe-Escherichia coli shuttle-Vektoren herzustellen.

Die Veröffentlichung "NTR1 encodes a higher affinity oligopeptide transporter in Avatidopsis von Doris Rentsch et. als (FEBS Letters 370 (1995) 264-268) zeigt eine heterologe Complementation von Hefe-Mutanten die 8 ermöglicht hat, Gene die für verschiedene Familien von Aminosäurentransportern kodieren zu isolieren.

In der Veröffentlichung von R. David et.al., Gene, 74 (1988) 527 - 574 wird die Konstruktion von neuen Allelen für Saccaromyces cerevisiae LEU2, U/2A send TRP1 Gene beschreiben.

Die WO 94/00574 offenbart eine DNA Sequenz, codierend für einen Oligosaccharid Transporter, dessen Einführung in ein Pflanzengenom die Bildung und den Transport von Speichermaterial in transgenen Pflanzen, Bakterien und Plasmiden modifiziert.

Es ist daher Aufgabe der Erfindung, ein Verfahren zur Erhöhung der Genexpression von Saccharose Synthase zu schaffen, durch das ein erhöhter Anteil an Enzym gebildet wird. Es ist ferner Aufgabe der Erfindung, Stoffe bereit zu stellen, die in einem solchen Verfahren einsetzbar sind.

Die Aufgabe wird erfindungsgemäß durch ein Verfahren gelöst, bei dem die Expression des Saccharose Synthasegens unter die Kontrolle eines Protonen-ATPase-Promotors gestellt wird. Dafür wird der Protonen-ATPase-Promotor dem Saccharose Synthasegen insbesondere vorgeschaltet. Das Gen stammt vorzugsweise aus Solanum tuberosum, während der Protonen-ATPase-Promotor vorzugsweise aus Hefe, insbesondere aus Saccharomyces cerevisiae stammt.

Eine weitere Erhöhung der Genexpression wird erzielt, indem die Kopienzahl des Saccharose Synthasegens und des Protonen-ATPase-Promotors erhöht wird. Dafür wird das Gen mit dem Promotor in ein Genkonstrukt, vorzugsweise in das Plasmid pDR195 (vgl. Figur 2) eingebaut und das Genkonstrukt anschließend in einen Mikroorganismus, insbesondere in den Hefestamm Saccharomyces cerevisiae 22574d (vgl. Jauniaux, J.-C. et al., Nitrogen catabolite regulation of proline permease in Saccharomyces cerevisiae. Cloning of the PUT4 gene and study of PUT4 RNA levels in wild-type and mutant strains, Eur. J. Biochem. 164, 601 - 606, 1987) transformiert.

Das rekombinante Enzym wird vorzugsweise nach Aufschluß der Zellen zusätzlich mittels Ionenaustausch und Ultrafiltration aufgereinigt. Da es für proteinchemische Anwendungen oft notwendig ist, sehr reines Protein bereit zu stellen, erfolgt für die weitere Reinigung der erwähnten technischen Enzympräparation daher insbesondere ein weiterer Reinigungsgang an Chelating Sepharose.

Die nach dem erfindungsgemäßen Verfahren erhältliche Saccharose Synthase ist für die Spaltung von Disacchariden, wie beispielsweise 2-Desoxysaccharose oder N-Acetylsaccharosamin, mit UDP verwendbar. Ebenso ist das Enzym für die Spaltung von Saccharose mit ADP verwendbar.

Das rekombinante Enzym ist generell genauso verwendbar wie das Enzym aus Reis (vgl. Patentschrift DE 42 21 595)

Die Erfindung wird im folgenden anhand eines Ausführungsbeispiels näher erläutert.

## Ausführungsbeispiel

### 1. Klonierung, Transformation und Expression der Saccharose Synthase aus Solanum tuberosum in Saccharomyces cerevisiae - Stamm 22574d.

Aus dem Plasmid 128A2SuSy (Figur 1) wurde das *sus1*-Gen mittels des Restriktionsenzyms BamHI herausgeschnitten. Die Schnittprodukte wurden elektrophoretisch in einem Agarosegel aufgetrennt, die *sus1*-Sequenz herausgeschnitten und eluiert.

Der Vektor pDR195 (Figur 2) wurde ebenfalls mit dem Restriktionsenzym BamHI geschnitten. Dieser Ansatz wurde anschließend durch eine Phenolisierung gereinigt, mit Ethanol gefällt, wieder aufgenommen und mit alkalischer Phosphatase dephosphoryliert. Die alkalische Phosphatase wurde innerhalb von 30 Minuten mit 50 mM EDTA bei 65°C desaktiviert. Es wurde erneut phenolisiert und anschließend mit Ethanol gefällt.

Mit ca. 200 µg Vektor und ca. 100 µg *sus1*-Sequenz wurde über Nacht bei 16°C mit T4-Ligase ligiert. Die Ligationsprodukte wurden in kompetente *E. coli*-Zellen, Stamm DH5α, transformiert und auf diese Weise vereinzelt und auf Selektivmedium vermehrt. Diese *E. coli*-Kolonien wurden im 3 ml-Maßstab angezogen und eine Plasmidpräparation daraus durchgeführt. Die Plasmide wurden jeweils mit den Restriktionsenzymen BamHI, BamHI+XhoI, HindIII geschnitten und die Schnittprodukte auf einem Agarosegel elektrophoretisch aufgetrennt. Mit Hilfe der sich ergebenden Bandenmuster konnten diejenigen Plasmidkonstrukte identifiziert werden, die die Sequenz *sus1* in der gewünschten Orientierung enthielten. Sie wurden als pDRSuSy bezeichnet (Figur 3). Ein zugehöriger *E. coli*-Stamm wurde ausgewählt und erneut im 3 ml-Maßstab angezogen, um hinreichend Plasmid für die Transformation der Hefezellen gewinnen zu können.

Zur Transformation der Hefezellen wurden zunächst kompetente Hefen erstellt. Dazu wurde nach dem folgenden, allgemeinen Protokoll vorgegangen:

Es wurden folgende Lösungen hergestellt und sterilisiert (Sterilfiltration empfohlen; es kann aber auch 15 Minuten bei 121°C autoklaviert werden):

Lösung A:
10 mM BICINE pH 8,35 (eingestellt mit KOH)
1 M Sorbit
3 % Ethylenglycol

Lösung B:
200 mM BICINE pH 8,35
40 % PEG 1000

Lösung C:
10 mM BICINE pH 8,35
150 mM NaCl

Die Hefezellen wurden über Nacht bei 30°C und 200 Umdrehungen pro Minute in 5 ml YPD-Medium angezogen:

| | | |
|---|---|---|
| 10 g | Hefeextrakt | |
| 20 g | Pepton | |
| 20 g | Glucose | auf 1 Liter dest. Wasser. |

Diese 5 ml-Kulturen wurden vollständig überführt in 200 ml YPD, die auf 30°C angewärmt wurden. Bei dieser Temperatur und 120 Umdrehungen pro Minute erreicht die Kultur nach ca. 3 Stunden eine $OD_{600}$ von 0,6. Die Kultur wurde 10 Minuten bei 4000 Umdrehungen pro Minute und 4°C abzentrifugiert und auf Eis mit 30 ml Lösung A gewaschen. Unter gleichen Bedingungen wurde erneut zentrifugiert und die Zellen in 2 ml Lösung A aufgenommen. Je 200 µl wurden in Reaktionsgefäße aliquotiert und im -70°C-Schrank eingefroren. Nach 60 Minuten sind sie weiter verwendbar und bleiben dies einige Monate lang.

Für die Transformation wurden 1-2 µg des Plasmids mit 50 µg Heringssperma-DNA in 10 µl Wasser gemischt und diese Lösung zu einem Aliquot der eingefrorenen Zellen gegeben. Diese wurden bei 37°C für 5 Minuten unter Schütteln getaut. Anschließend wurde 1 ml Lösung B zugegeben und der Ansatz unter vorsichtigem Schütteln bei 30°C 60 Minuten lang inkubiert. Danach wurden die Zellen für 2 Minuten bei 3000 Umdrehungen pro Minute zentrifugiert, das Pellet mit 500 µl Lösung C gewaschen, erneut zentrifugiert und in 100 µl Lösung C aufgenommen. Diese Suspension wurde auf SD-Festmedium mit 2 % Glucose ausplattiert.

Die so mit Plasmid pDRSuSy transformierten Hefen des Stammes 22574d wurden 2 Tage bei 30°C auf Platten wachsen gelassen. Die sich zeigenden Kolonien wurden auf SD-Medium weitervermehrt. Außerdem wurden diese Kolonien in flüssigem SD-Medium bis in die stationäre Phase angezogen (30°C, 120 Umdrehungen pro Minute Schüttelgeschwindigkeit), die Zellen aufgeschlossen und der sich ergebende Extrakt im Vergleich mit dem Extrakt aus nicht transformierten Zellen auf einem 10%igen SDS-Polyacrylamidgel elektrophoretisch aufgetrennt. Parallel wurde der Extrakt mit einem Enzymtest vermessen (Elling, L. und Kula, M.-R.: Purification of sucrose synthase from rice and its

protein-chemical characterization, in: J. Biotechnol. 29, 277 - 286, 1993).

Das Polyacrylamidgel lieferte eine 90.000 kD Bande bei den transformierten Zellen, die bei den nicht transformierten fehlte. Eine Bande dieser Größe wurde für das Monomer der Saccharose Synthase erwartet. Der Enzymtest lieferte eine eindeutige Aktivität des Extrakts der transformierten Zellen. Demgegenüber war die Aktivität der nichttransformierten Zellen unterhalb der Nachweisgrenze.

Da die Zellen das Gen konstitutiv exprimieren, muß kein besonderer Erntezeitpunkt beachtet werden. Für den 10 l-Anzuchtmaßstab wird wie folgt vorgegangen: Von den monatlich neu auszustreichenden Erhaltungskulturen wird eine Kolonie ausgewählt und in ein Röhrchen mit 3 ml SD - 2 % Glucose überführt. Nach ca. 24 Stunden ist die Kultur ausgewachsen (alle Angaben beziehen sich auf 30°C Temperatur und 120 Umdrehungen pro Minute Schüttelgeschwindigkeit). Die Kultur wird in 50 ml des gleichen Mediums überführt und erneut 24 Stunden inkubiert. Anschließend wird die 50 ml-Kultur in 250 ml überführt und wiederum 24 Stunden geschüttelt. Mit den 250 ml impft man schließlich 5 mal 2 l SD - 2 % Glucose an und läßt die Kulturen zu Ende wachsen (über Nacht zu einer $OD_{600}$ von ca. 3,5 bis 4,0).

Die Zellen werden per Zentrifugation geerntet, in 200 mM HEPES pH 7,6 im Verhältnis 4:6 (w/w) aufgenommen und können dann direkt aufgeschlossen oder einige Monate bei -20°C gelagert werden.

**2. Aufreinigung der rekombinanten Saccharose Synthase**

Die in 200 mM HEPES pH 7,6 aufgenommenen Zellen wurden in einer Glasperlenmühle 20 Minuten bei 4000 Umdrehungen pro Minute Rührgeschwindigkeit zermahlen. Die verwendeten Glasperlen hatten einen Durchmesser von 0,5 mm. Während und nach dem Mahlvorgang wurde die Suspension auf Eis gehalten. Abschließend wurden die festen Bestandteile durch eine Zentrifugation bei 15000 Umdrehungen pro Minute und 4°C über 15 Minuten abgetrennt.

Die Saccharose Synthase wurde anschließend mittels Anionenaustauschchromatographie aufgereinigt. Die Chromatographieanlage bestand aus einer Säule mit 300 ml Q-Sepharose FF, einer P1-Pumpe, einem Detektor UV-1 (280 nm), einem Fraktionssammler Frac 300 sowie einem Schreiber REC 101. Alle Materialien stammten von Pharmacia.

Die Säule wurde mit 1 Liter 50 mM HEPES-NaOH pH 8,0 (Standardpuffer) bei einer Flußrate von 10 ml pro Minute äquilibriert. Die Flußgeschwindigkeit blieb für alle weiteren Schritte konstant.

Anschließend erfolgte die Ladung von Zentrifugationsüberstand, wobei Material aus bis zu 5 Litern Kultur aufgegeben werden kann. Nicht gebundene Proteine wurden mit 1 Liter Standardpuffer mit 0,1 M KCl ausgewaschen.

Der Salzgradient wurde mit Puffer A (Standardpuffer mit 0,1 M KCl) und Puffer B (Standardpuffer mit 0,4 M KCl) gestartet. Das Gesamtvolumen des Gradienten betrug 1 Liter, kann aber auch auf 1,5 Liter gestreckt werden. Im Bereich zwischen 0,2 und 0,3 M KCl eluiert die Saccharose Synthase.

Mit dem folgenden Spülschema wurde die Säule regeneriert: 500 ml 0,1 M Kaliumacetat pH 4,0 mit 1 M NaCl; 1 Liter Wasser; 300 ml 2 M NaOH; 1 Liter Wasser; 500 ml 50 mM HEPES pH7,6 mit 1 M NaCl; 1 Liter Wasser.

Zur Lagerung bei 4°C wurde das Gel in 20 % Ethanol eingelegt.

Die Saccharose Synthase enthaltenden Fraktionen wurden vereinigt und sukzessive an einer 50 ml Ultrafiltrationszelle der Firma Amicon eingeengt. Als Membran diente eine Amicon YM30 (Ausschlußgröße 30000d), Durchmesser 43 mm.

Pro Lauf einer 300 ml Q-Sepharose FF-Säule kann auf 5 bis 10 ml eingeengt werden.

Die erhaltene Präparation ist für Synthesen verwendbar.

Für Untersuchungen des Proteins selbst wurde anschließend die rekombinante Saccharose Synthase einer weiteren Reinigung an Chelating Sepharose unterworfen. Dafür wurde das Gelmaterial "Chelating Sepharose Fast Flow" von Pharmacia verwendet.

Die mit 50 ml Gelmaterial gepackte Säule wurde mit 3 Volumen 1 M NaCl in 0,1 M Natriumacetat pH 4,0 äquilibriert. Anschließend wurden im gleichen Puffer 0.1 M $CuSO_4$ gelöst und so lange über die Säule gespült, bis die Säule gleichmäßig blau gefärbt war. Überschüssiges Kupfersulfat wurde mit 3 Säulenvolumen 1 M NaCl in 0,1 M Natriumacetat pH 4,0 heruntergespült.

Vor Probenaufgabe wurde die Säule wie folgt äquilibriert: Es wurde zunächst mit 150 mM KCl in 200 mM HEPES pH 7,2, anschließend mit je 3 Säulenvolumen dieses Puffers und 10 mM Imidazol und schließlich mit diesem Puffer und 1 mM Imidazol gespült.

Die Probe bzw. Proteinlösung wurde zunächst auf eine Konzentration von 1 mM Imidazol gebracht und anschließend über die Säule gepumpt. Ungebundene Proteine wurden von der Säule mit 1 mM Imidazol in 150 mM KCl und 200 mM HEPES pH 7,2 gewaschen. Die allmähliche Elution der gebundenen Proteine erfolgte über einen Imidazolgradienten von 1 mM bis 70 mM in 150 mM KCl und 200 mM HEPES pH 7,2. Dabei erscheinen die meisten Proteine bei 20 bis 30 mM Imidazol, während die Saccharose Synthase bei 30 bis 55 mM erscheint.

Mit Hilfe eines anschließenden Waschschrittes mit 100 mM Imidazol in 150 mM KCl und 200 mM HEPES pH 7,2 wurde die Säule in den Zustand der erneuten Benutzbarkeit gebracht. Ist eine weitere Nutzung nicht gewünscht, wird das Kupfer mit 3 Säulenvolumen 10 mM EDTA in 150 mM KCL und 200 mM HEPES pH 7,2 heruntergewaschen.

Zur vollständigen Reinigung erfolgt eine weitere Waschung mit 3 Säulenvolumen 50 mM EDTA in Wasser. Damit ist das Material wieder in seinem Ausgangszustand. Es wird in 20 % Ethanol bei 4°C gelagert.

Mit Hilfe dieses Reinigungsganges gelang die Weiterreinigung einer Präparation von 2,4 U Saccharose Synthase / mg Protein auf 6,4 U / mg Protein bei einer Ausbeute von 82 %. Das entspricht einem Reinigungsfaktor von 2,7. Insgesamt konnten im Eluat 96 % der eingesetzten Aktivität wiedergefunden werden.

Tabelle 1 zeigt einen Vergleich der Reinigungsschemen von Hefe und Reis. Die folgenden Schritte der Ionenaustauschchromatographie und Ultrafiltration erfolgen bei Hefe und Reis identisch: die Chromatographie an Q-Sepharose FF, die Ultrafiltration an Membranen mit einem cut-off von 30000 Da. Die Reispräparation muß anschließend noch auf einer Gelfiltrationssäule feingereinigt werden, um noch vorhandene Invertase abzutrennen, was allerdings nicht vollständig gelingt. Dies führt bei der Applikation der Saccharose Synthase in Synthesen zu unerwünschten Abbaureaktionen der Produkte. Demgegenüber enthält Hefe keine Invertase, wenn man sie auf saccharosefreiem Medium anzieht. Eine Feinreinigung per Gelfiltration ist daher nicht notwendig. Dies spart Material, beschleunigt den Reinigungsprozeß und erhöht die Ausbeute an Enzym, stellt also einen ökonomischen Vorteil dar.

Tabelle 2 zeigt einen Vergleich zweier Aufarbeitungen aus Hefe und Reis nach dem Reinigungsschema von Tabelle 1: Die in der Biomasse vorhandenen Aktivität ist in der Hefe um den Faktor 10 höher als im Reis. Entsprechend höher ist auch die spezifische Aktivität des Enzyms im Rohaufschluß. Da die in den folgenden Aufarbeitungsschritten benutzten Säulen in ihrer Beladungskapazität durch die Menge an aufgegebenem Protein begrenzt sind, kann daher pro Säulenlauf eine größere Menge Enzym gereinigt werden.

Nach Ionenaustauscher und Ultrafiltration ist das rekombinante Enzym aus Hefe sauberer als das Enzym aus Reis. Wichtiger ist jedoch, daß die Präparation des rekombinanten Enzyms keine relevanten Mengen an Nebenaktivitäten mehr enthält. Für das Enzym aus Hefe ist die Reinigung damit bei einer Gesamtausbeute von 40% abgeschlossen. Demgegenüber muß die Reispräparation noch einem Gelfiltrationsschritt unterworfen werden, der die Sauberkeit um den Faktor 10 erhöht, aber die Nebenaktivitäten nur ungenügend abtrennen kann. Statt dessen sinkt die Ausbeute der Gesamtreinigung auf 11,3%.

Tabelle 3 vergleicht die Nebenaktivitäten in den beschriebenen Enzympräparationen aus Hefe und Reis. Wie in Tabelle 1 dargestellt, umfaßt die Reinigung des rekombinanten Enzyms aus Hefe 4 Schritte, während die Reinigung aus Reis 5 Schritte umfaßt. In beiden Fällen können die NDP abbauenden Phosphatasen komplett entfernt werden. Die in der Hefe aufgrund der Kulturbedingungen nicht vorhandene Invertase ist in der Präparation aus Reis noch in solchen Mengen vorhanden, daß einige langsame Synthesereaktionen stark gestört werden, da die Invertase das Syntheseprodukt sogleich wieder spaltet. UDP-Glucose abbauende Aktivitäten treten in der Präparation aus Hefe in viel geringerem Maße auf.

### 3. Saccharose Spaltung mit der rekombinanten Saccharose Synthase

Es wurde zunächst die Akzeptanz der rekombinanten Saccharose Synthase für unterschiedliche NDPs zur Saccharose Spaltung untersucht. Die Reaktionsbedingungen für diese Untersuchung waren wie folgt:

| | |
|---|---|
| NDP-Konzentration | 1,6 mM |
| Saccharose | 500 mM |
| Saccharose Synthase | 0,035 U |
| Puffer (HEPES-KOH, pH 7,6) | 200 mM |
| Volumen | 1 ml |

Die Reaktionen mit den unterschiedlichen NDPs wurden bei einer Temperatur von 30°C durchgeführt.

Die Ergebnisse sind in Figur 4 dargestellt. Als Akzeptanz von NDPs zur Saccharose Spaltung in Abhängigkeit von der Reaktionszeit ergibt sich die folgende Reihe:

UDP > ADP = TDP > CDP > GDP

Desweiteren wurde mit der rekombinanten Saccharose Synthase die Michaelis-Menten-Kinetik der Saccharose Spaltung mit UDP in Abhängigkeit zur Saccharosekonzentration bestimmt. Die Reaktionsbedingungen dafür waren wie folgt:

| | |
|---|---|
| UDP-Konzentration | 1,6 mM |
| Saccharose Synthase | 14 mU |

(fortgesetzt)

| | |
|---|---|
| Puffer (HEPES-KOH, pH 7,6) | 200 mM |
| Volumen | 1 ml |

Die Reaktionen wurden bei einer Temperatur von 30°C durchgeführt; die Reaktionszeit betrug jeweils 10 Minuten.

In Figur 5 ist die Michaelis-Menten-Kinetik der Saccharose Spaltung in Abhängigkeit zur Saccharosekonzentration dargestellt. Die Auftragung zeigt einen Km für Saccharose von 66 mM. Bei Konzentrationen von mehr als 600 mM Saccharose kommt es zu einer Substratüberschußhemmung.

**4. Synthese ausgewählter Nucleotidzucker mit der rekombinanten Saccharose Synthase**

Die im folgenden beschriebenen Synthesen von ADP-Glucose, UDP-2-Desoxyglucose und UDP-N-Acetylglucosamin folgen der Spaltrichtung des Enzyms mit ADP bzw. UDP.

4.1 Synthese von ADP-Glucose

Für die Synthese von ADP-Glucose wurde folgender Reaktionsansatz verwendet:

| | |
|---|---|
| AMP | 4 mM |
| ATP | 4 mM |
| Rekombinante SaccharoseSynthase | 100 U |
| Myokinase aus Kaninchenmuskel | 10 U |
| BSA | 100 mg |
| $MgCl_2$ | 0,125 |
| Puffer A | 100 ml |

| | | |
|---|---|---|
| Puffer A | HEPES-NaOH, pH 7,5 | 200 mM |
| | Saccharose | 500 mM |
| | DTT | 3 mM |

Der Reaktionsansatz wurde sterilfiltriert und anschließend über Nacht bei 30°C gerührt. Am nächsten Tag wurde die Reaktionslösung in einer Amiconzelle Typ 8050 mit einer YM 10-Membran (cut-off von 10.000 d) auf 10 ml eingeengt. Dadurch blieben die Proteine im Ansatz, während die Reaktionsprodukte abgezogen wurden.

Mit 90 ml der folgenden Substratlösung:

| | |
|---|---|
| AMP | 4 mM |
| ATP | 4 mM |
| $MgCl_2$ | 0,125 mM |
| in Puffer A, sterilfiltriert, | |

wurde der Ansatz aufgefüllt und eine erneute Reaktion über Nacht durchgeführt. Diese wurde insgesamt 10 mal wiederholt. Im Verlauf der Reaktionswiederholung wurden 10 U Myokinase nachdosiert.

Auf diese Weise waren 2,8 g ADP-Glucose mit einer Ausbeute von 55% in Bezug auf die eingesetzten AMP und ATP herstellbar. Die Gesamtausbeute der Synthese nach erfolgter Reinigung betrug 2,2 g entsprechend 43,6%.

4.2 Synthese von UDP-2-Desoxyglucose und UDP-N-Acetylglucosamin

Für die Synthese von UDP-2-Desoxyglucose und UDP-N-Acetylglucosamin wurde folgender Reaktionsansatz verwendet:

| | |
|---|---|
| Disaccharid | 250 mM |
| UDP | 2 mM |

(fortgesetzt)

| | |
|---|---|
| Saccharose Synthase | in unterschiedlichen Mengen |
| HEPES-NaOH, pH 7,6 | 200 mM |
| Volumen | 1 ml |

Die Reaktionsansätze wurden 24 Stunden bei 30°C inkubiert und die Reaktionen anschließend bei 95°C über 5 Minuten gestoppt.

Auf diese Weise waren mit 0,11 U Saccharose Synthase 5,9% der 2-Desoxysaccharose spaltbar. Mit 1,25 U des Enzyms konnten 12,4% des N-Acetylsaccharosamins umgesetzt werden.

Tabelle 1:

| Vergleich der Reinigungsschemen von Hefe und Reis | |
|---|---|
| Hefe | Reis |
| Aufschluß<br>Zentrifugation<br>Ionenaustausch<br>Ultrafiltration<br>/ | Aufschluß<br>Filtration<br>Ionenaustausch<br>Ultrafiltration<br>Gelfiltration |

Tabelle 2:

| Vergleich der Aufarbeitungsdaten von Hefe und Reis | | |
|---|---|---|
| | Hefe | Reis |
| Aktivität pro Gramm Zellen bzw. Reissamen | 6,2U/g | 0,56U/g |
| spezifische Aktivität im Rohaufschluß | 0,22U/mg Protein | 0,07U/mg Protein |
| spezifische Aktivität nach Ionentauscher und Ultrafiltration | 2,4U/mg Protein | 1,4U/mg Protein |
| spezifische Aktivität zu Reinigungsabschluß | 2,4U/mg Protein | 13,6U/mg Protein |
| Ausbeute Gesamtreinigung | 40% | 11,3% |

Tabelle 3:

| Vergleich der Nebenaktivitäten | | |
|---|---|---|
| Nebenaktivitäten | Hefe aus 4 Schritten | Reis aus 5 Schritten |
| Phosphatasen | 0 | 0 |
| Invertase | 0 | 0,05% |
| UDPGlucose Abbau | 0,0018% | 0,05% |

**Patentansprüche**

1. Verfahren zur Erhöhung der Genexpression von Saccharose Synthase
**dadurch gekennzeichnet,**
**dass** die Expression der Saccharose Synthase aus dem Saccharose Synthasegen aus Solanum tuberosum unter der Kontrolle eines Protonen-ATPase Promotors aus Saccharomyces cerevisiae erfolgt, welche in ein Genkonstrukt eingebaut sind, mit dem Saccharomyces cerevisiae transformiert wird.

2. Verfahren nach Anspruch 1,
**dadurch gekennzeichnet,**
**dass** dem Saccharose Synthasegen der Protonen ATPase-Promotor vorgeschaltet wird.

3. Verfahren nach einem der Ansprüche 1 oder 2,
**dadurch gekennzeichnet,**
**dass** die Genexpression der Saccharose Synthase zusätzlich durch Erhöhen der Kopienzahl des Saccharose Synthasegens und des Protonen-ATPase-Promotors erhöht wird.

4. Verfahren nach einem der vorhergehenden Ansprüche,
**dadurch gekennzeichnet,**
**dass** die Saccharose Synthase zusätzlich aufgereinigt wird.

5. Verfahren nach Anspruch 4,
**dadurch gekennzeichnet,**
**dass** ein Reinigungsschritt eine Ionenaustauschchromatographie umfasst.

6. Verfahren nach Anspruch 5,
**dadurch gekennzeichnet,**
**dass** die Ionenaustauschchromatographie eine Anionenaustauschchromatographie ist.

7. Verfahren nach einem der Ansprüche 4 bis 6,
**dadurch gekennzeichnet,**
**dass** ein Reinigungsschritt eine Ultrafiltration umfasst.

8. Verfahren nach einem der Ansprüche 4 bis 7,
**dadurch gekennzeichnet,**
**dass** zur Feinreinigung des Enzyms ein Reinigungsgang an Chelating Sepharose erfolgt.

9. Saccharose Synthasegen aus Solanum tuberosum mit einem vorgeschalteten Protonen-ATPase-Promotor aus Saccharomyces cerevisiae.

10. Genstruktur, enthaltend ein Saccharose Synthasegen nach Anspruch 9.

11. Vektor enthaltend ein Saccharose Synthasegen nach Anspruch 9 oder eine Genstruktur nach Anspruch 10.

12. Transformierte Zelle, enthaltend in replizierbarer Form ein Saccharose Synthasegen nach Anspruch 9.

13. Transformierte Zelle nach Anspruch 12, enthaltend eine Genstruktur nach Anspruch 10 oder einen Vektor nach Anspruch 11.

14. Transformierte Zelle nach Anspruch 12 oder 13,
**dadurch gekennzeichnet,**
**dass** sie eine Hefezelle ist.

15. Transformierte Zelle nach Anspruch 14,
**dadurch gekennzeichnet,**
**dass** sie Saccharomyces cerevisiae ist.

## Revendications

1. Procédé pour augmenter l'expression génique de la saccharose synthase
**caractérisé**
**en ce que** l'expression de la saccharose synthase à partir du gène de la saccharose synthase provenant de solanum tuberosum s'effectue sous le contrôle d'un promoteur de proton-ATPase provenant de saccharomyces cerevisiae, qui est incorporé dans un assemblage de gène, par lequel saccharomyces cerevisiae est transformé.

2. Procédé suivant la revendication 1
**caractérisé**
**en ce que** le promoteur de proton-ATPase est en amont du gène de la saccharose synthase.

3. Procédé suivant l'une des revendications 1 ou 2 **caractérisé** **en ce que** l'expression génique de la saccharose synthase est augmentée en outre en augmentant le nombre de copies du gène de la saccharose synthase et du promoteur de la proton-ATPase.

4. Procédé suivant l'une des revendications précédentes **caractérisé** **en ce que** la saccharose synthase est en outre purifiée.

5. Procédé suivant la revendication 4 **caractérisé** **en ce qu'**un stade de purification comprend une chromatographie avec échange d'ions.

6. Procédé suivant la revendication 5 **caractérisé** **en ce que** la chromatographie avec échange d'ions est une chromatographie avec échange d'anions.

7. Procédé suivant l'une des revendications 4 à 6 **caractérisé** **en ce qu'**un stade de purification comprend une ultrafiltration.

8. Procédé suivant l'une des revendications 4 à 7 **caractérisé** **en ce que** pour purifier finement l'enzyme une opération de purification sur sépharose de chélation est effectuée.

9. Gène de la saccharose synthase provenant de solanum tuberosum ayant un promoteur de proton-ATPase en amont provenant de saccharomyces cerevisiae.

10. Structure de gène contenant un gène de saccharose synthase suivant la revendication 9.

11. Vecteur contenant un gène de saccharose synthase suivant la revendication 9 ou une structure de gène suivant la revendication 10.

12. Cellule transformée contenant sous une forme réplicable un gène de saccharose synthase suivant la revendication 9.

13. Cellule transformée suivant la question 12 contenant une structure de gène suivant la revendication 10 ou un vecteur suivant la revendication 11.

14. Cellule transformée suivant la revendication 12 ou 13 **caractérisée** **en ce que** c'est une cellule de levure.

15. Cellule transformée suivant la revendication 14 **caractérisée** **en ce que** c'est saccharomyces cerevisiae.

## Claims

1. Method for increasing the gene expression of saccharose synthase, **characterised in that** the expression of the saccharose synthase from saccharose synthase gene of solanum tuberosum is carried out under control of a proton ATPase promotor of saccharomyces cerevisiae which are incorporated in a gene structure by means of which saccharomyces cerevisiae is transformed.

2. Method according to Claim 1, **characterised in that** the proton ATPase promotor is ahead of the saccharose synthase gene.

3. Method according to one of Claims 1 or 2, **characterised in that** the gene expression of the saccharose synthase is additionally increased by increasing the copy number of the saccharose synthase gene and the proton ATPase promotor.

4. Method according to one of the above claims, **characterised in that** the saccharose synthase is additionally purified.

5. Method according to Claim 4, **characterised in that** a purification stage includes an ion exchange chromatography.

6. Method according to Claim 5, **characterised in that** the ion exchange chromatography is an anion exchange chromatography.

7. Method according to one of Claims 4 to 6, **characterised in that** a purification stage includes ultra filtration.

8. Method according to one of Claims 4 to 7, **characterised in that** a purification stage on chelating sepharose is carried out for fine purification of the encyme.

9. Saccharose synthase gene of solanum tuberosum with a leading proton-ATPase promotor of saccharomyces cerevisiae.

10. Gene structure contqining a saccharose synthase gene according to Claim 9.

11. Vector containing a saccharose synthase gene according to Claim 9 or a gene structure according to Claim 10.

12. Transformed cell containing a replicable form of a saccharose synthase gene according to Claim 9.

13. Transformed cell according to Claim 12, containing a gene structure according to Claim 10 or a vector according to Claim 11.

14. Transformed cell according to Claim 12 or 13, **characterised in that** it is a yeast cell.

15. Transformed cell according to Claim 14, **characterised in that** it is saccharomyces cerevisiae.

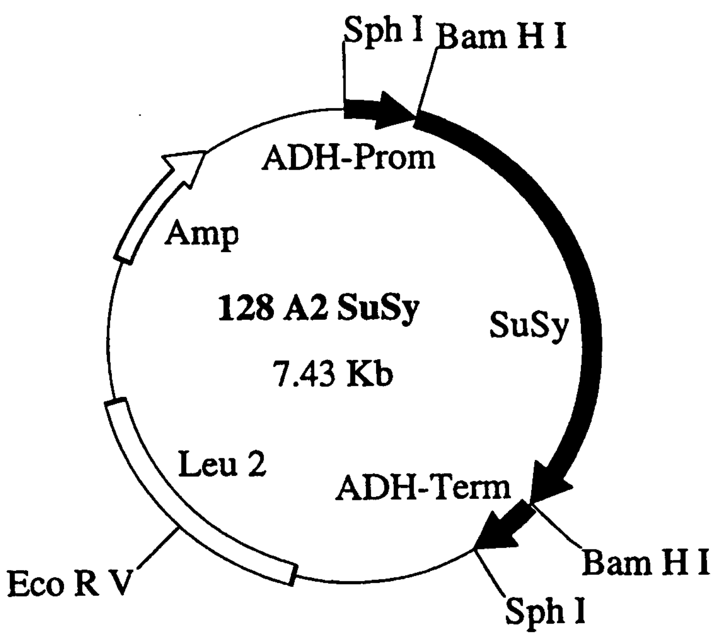
Sph I
Bam H I
ADH-Prom
Amp
128 A2 SuSy
7.43 Kb
SuSy
Leu 2
ADH-Term
Eco R V
Bam H I
Sph I

Fig. 1

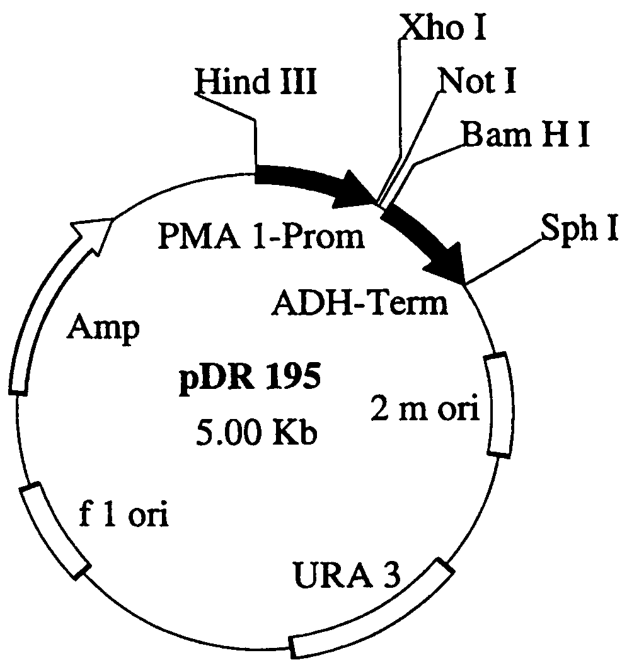
Xho I
Hind III
Not I
Bam H I
Sph I
PMA 1-Prom
ADH-Term
Amp
pDR 195
5.00 Kb
2 m ori
f 1 ori
URA 3

Fig. 2

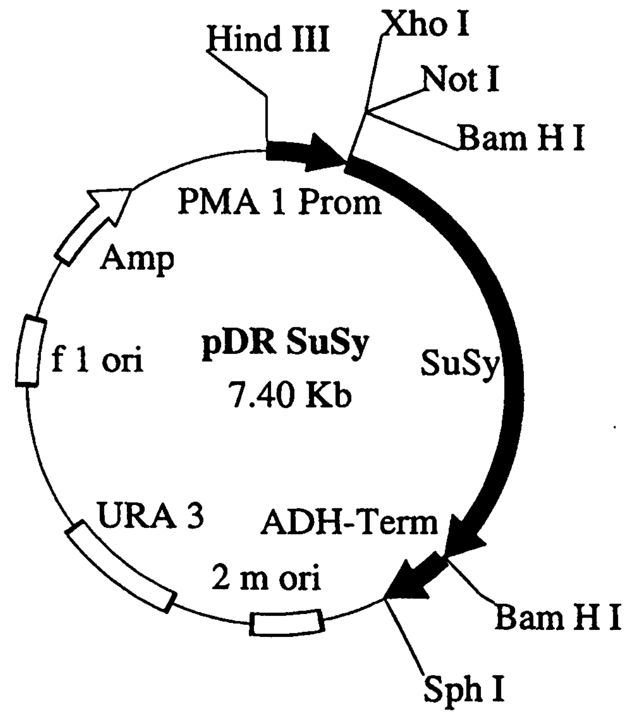
Hind III
Xho I
Not I
Bam H I
PMA 1 Prom
Amp
f 1 ori
pDR SuSy
7.40 Kb
SuSy
URA 3
ADH-Term
2 m ori
Bam H I
Sph I

Fig. 3

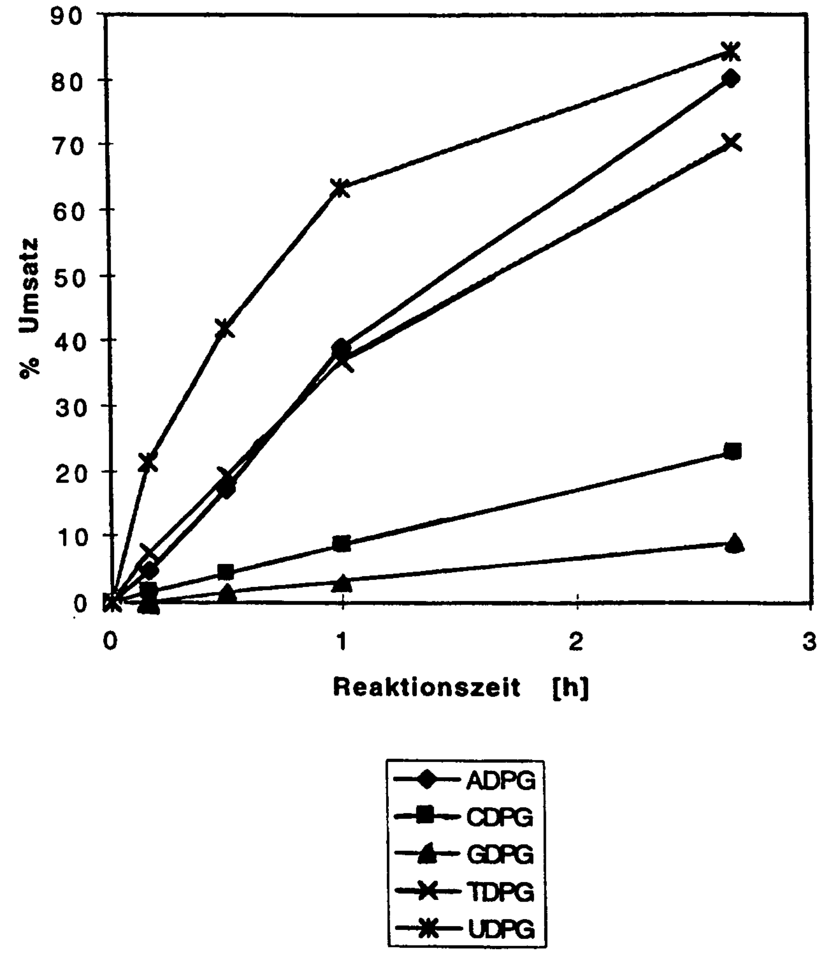
90
80
70
60
50
40
30
20
10
0
% Umsatz
0
1
2
3
Reaktionszeit [h]
ADPG
CDPG
GDPG
TDPG
UDPG

Fig. 4

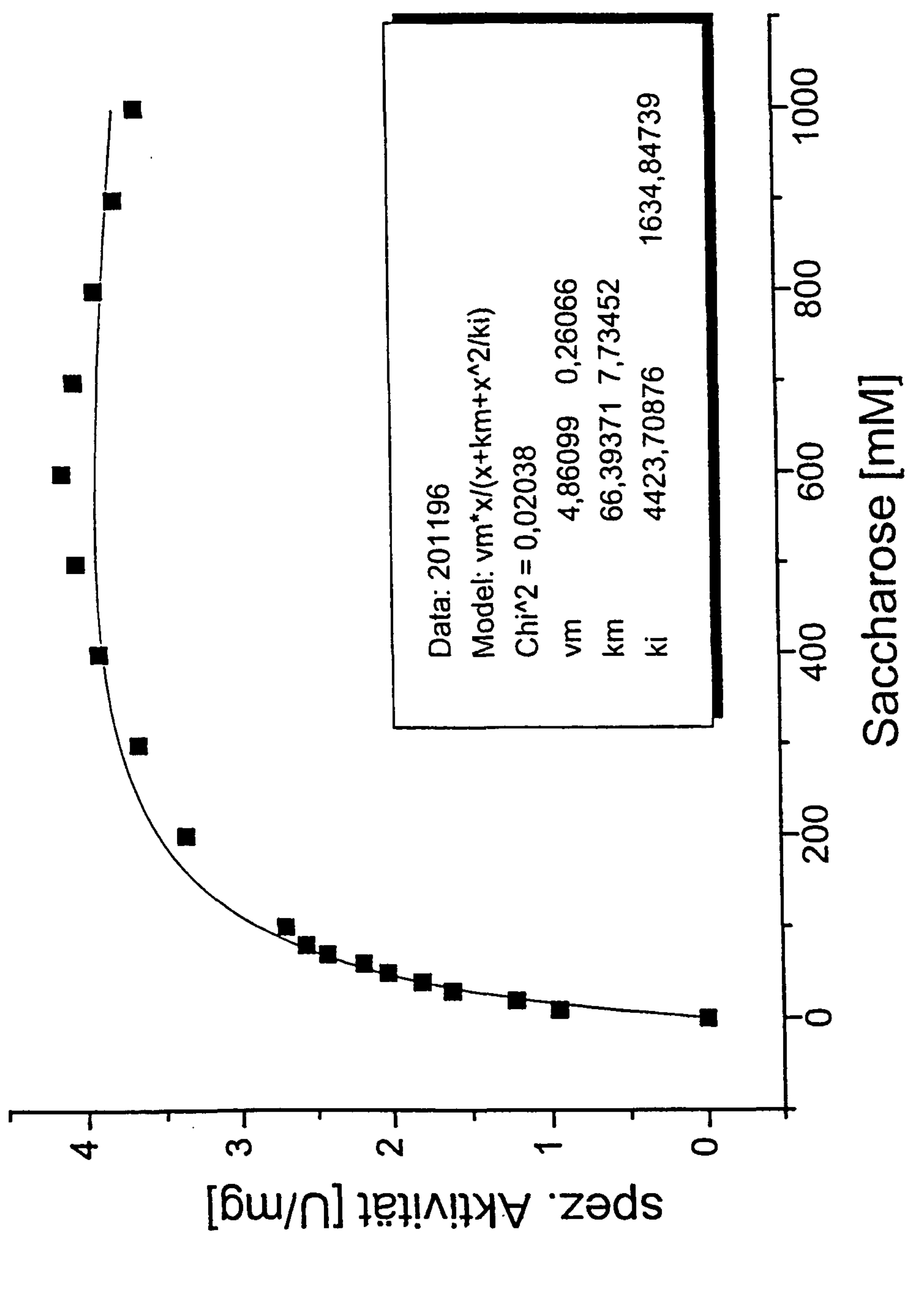
Data: 201196
Model: vm*x/(x+km+x^2/ki)
Chi^2 = 0,02038
vm 4,86099 0,26066
km 66,39371 7,73452
ki 4423,70876 1634,84739
spez. Aktivität [U/mg]
Saccharose [mM]
0
1
2
3
4
0
200
400
600
800
1000

Fig. 5